# EUROPEAN PATENT APPLICATION

(11) **EP 4 782 542 A1**
(43) Date of publication of application: **29.07.2026**
(21) Application number: 24866810.5
(22) Date of filing: 29.03.2024
(51) Int. Cl.: C12N 15/70, C12N 15/62, C07K 14/435, C07K 14/245

(54) **SYNTHETIC ORGANELLE-BASED METHOD FOR RECOVERING TARGET PRODUCT, DNA CONSTRUCT AND EXPRESSION SYSTEM**

(30) Priority: 22.09.2023 CN 202311244877; 13.11.2023 CN 202311503445
(71) Applicant: Ailurus Ltd, Edinburgh, EH6 7BD (GB)
(72) Inventor: GUO, Haotian, London (GB)
(74) Representative: Finnegan Europe LLP
(86) International application number: PCT/CN2024/084910
(87) International publication number: WO 2025/060384

(57) **Abstract**

A synthetic organelle-based method for recovering a target product, a DNA construct and an expression system. Specifically, the method comprises: constructing a synthetic organelle in a host; collecting a target product using the synthetic organelle; recovering the synthetic organelle containing the target product; and releasing the target product from the synthetic organelle containing the target product to obtain the target product. Provided is a universal method for product recovery and/or purification, which has the advantages of simple steps, low cost, convenient implementation for scale-up and the capability of performing high-throughput parallel treatment.

## Description

### Technical Field

The present disclosure relates to a method for recovering a target product based on a synthetic organelle, a DNA construct, and an expression system. More specifically, the present disclosure relates to a method for separation, recovery, and purification of a target product by constructing synthetic organelles in a host and controlling the process of collecting and releasing the target product by the synthetic organelle.

### Background Art

Currently, the separation, recovery, and purification of various target products require complex processes. These processes often need customized arrangement and combination of various unit operations in chemical engineering, such as extraction, chromatography, electrophoresis, centrifugation, and membrane treatment, according to the physical, chemical, and biological properties of the target products. Although these processes can efficiently and specifically recover and purify products, they generally have defects such as cumbersome steps, long time consumption, and expensive equipment and consumables. Moreover, these processes lack universality, making it difficult to perform high-throughput parallel processing for separation, recovery, and purification of different products.

For target products that can be artificially modified, such as recombinant proteins, affinity chromatography can be used for their purification by connecting affinity tags. Although such processes can be general to an extent, they involve tag removal and secondary purification, resulting in complex steps and still high costs. The affinity tags suitable for different target proteins are not completely consistent, and may need different chromatographic column media. Since different target proteins exhibit different migration speeds in the mobile phase, the flow rates used in the chromatography process and the durations for steps will also have considerable differences. Therefore, affinity chromatography purification methods for recombinant proteins based on affinity tags are also difficult to operate in parallel.

Some target products with special properties can use simple and low-cost recovery methods. For example, if a target product has high solubility in a specific solvent, the target product can be enriched in that solvent and then separated and recovered. If a target product is a certain natural organelle in a cell, gradient density centrifugation can be performed after cell disruption to obtain that organelle. Further, if a target product is a certain molecule in a natural organelle, such as the mitochondrial genome, the organelle can be separated first and then disrupted to recover the target product. However, the process development difficulty of these methods is high, and it is difficult to apply them to the recovery and purification of other products.

CN112575016A discloses "Construction of Membraneless Organelles in Prokaryotes and Use thereof", which successfully constructed membraneless compartments in *E. coli* using recombinant spider fibroin and arthropod elastin-like protein, and achieved intracellular co-localization of target functional proteins by connecting spider fibroin or arthropod elastin-like protein to different cargo proteins through DNA recombination technology, thereby constructing membraneless compartments with biological activity. This method directly expresses fusion proteins. Although it has achieved some success to a certain extent and can form an enriched phase of cargo proteins in cells, after the host cells are disrupted, this structure is disassembled. Therefore, this structure lacks the spatial independence that organelles should have, making it not separable and recoverable. In addition, the design of directly expressing fusion proteins also lead to low activity of the connected cargo proteins and restricted specific expression of assembly proteins.

WO2023015190A1 discloses a method for controlling cellular processes in mammalian cells through synthetic organelles, which uses an arginine/glycine-rich (RGG) protein domain capable of liquid-liquid phase separation (LLPS) connected with a high-affinity coiled-coil tag to enrich endogenous proteins also connected with a coiled-coil tag, thereby controlling cell behavior by isolating or releasing endogenous proteins. This method respectively adds tags to the RGG protein forming the organelle and the target protein, better ensuring the correct folding and activity of the target protein. However, since the goal of this method is to control mammalian cell behavior, both the recruitment and release of target proteins by synthetic organelles must occur within the cell. Therefore, this method does not allow experimental steps for separating and recovering synthetic organelles from host cells.

Therefore, there is currently a need for a technology that can separate and recover synthetic organelles and utilize them.

### Summary of the Invention

To solve the above problems, the present disclosure provides a method for recovering a target product based on a synthetic organelle, which achieves separation, recovery, and purification of the target product by constructing a synthetic organelle in a host and using the synthetic organelle to collect and release the target product.

Specifically, if a synthetic organelle can be constructed in a host for product recovery, and if the target product can enter the synthetic organelle or the target product *per se* is produced in the synthetic organelle, then the synthetic organelle can be used to collect the target product, subsequently be recovered, and finally be placed in an eluent to release the target product, thereby allowing recovery and/or purification of the product. On this basis the present disclosure is completed.

The synthetic organelles that can be used in the present disclosure can be either artificially synthesized membrane-bound organelles or artificially synthesized membraneless organelles. Preferably, the host is a cellular system, and the synthetic organelle is a membraneless organelle. Membraneless organelles are biomolecular condensates with obvious spatial independence formed by biomacromolecules through phase separation. The synthetic biomacromolecules that can be used to construct membraneless organelles in the present disclosure include nucleotide sequences with 10-1000 consecutive repeats of nucleotide repeat units, such as CAG triplets, CCUG, GGGAA, GGGGCC, etc.; amino acid sequences with 3-500 consecutive repeats, such as repeats of resilin-like peptides (RLP); intrinsically disordered regions, such as FUS protein, ParB protein; polymerization of multivalent biomacromolecules, such as PTB protein with UCUCU sequence repeats, artificially designed binary protein two-dimensional structures, and artificially designed binary RNA two-dimensional structures.

The target products that can be used in the present disclosure can be any molecule or structure intended to be prepared, including samples obtained from the external environment or products synthesized or produced biologically by an artificially modified host. The target products and synthetic organelles can be co-generated in the same host cell, or separately in different hosts. Preferably, the biological synthesis route of at least one component of the target product is encoded by a heterologous DNA, the component including: heterologously biosynthesized small molecules, such as deoxyviolacein; recombinantly expressed polypeptides, proteins, RNA, and DNA, such as green fluorescent protein, red fluorescent protein, T4 DNA ligase, 16S rRNA; and multi-component complexes, such as 30S ribosomal subunits, CRISPR-Cas complexes with RNA.

In one aspect, the present disclosure provides a method for recovering a target product based on a synthetic organelle, comprising:
a. constructing a synthetic organelle in a host;
b. using the synthetic organelle to collect a target product;
c. recovering the synthetic organelle containing the target product;
d. releasing the target product from the synthetic organelle containing the target product, thereby obtaining the target product.

In one embodiment, the synthetic organelle refers to an artificially designed compartmentalized structure, preferably, the synthetic organelle refers to a membraneless organelle, which is a biomolecular condensate formed in a host by polypeptides and/or proteins and/or DNA and/or RNA and/or nucleic acid-protein complexes, more preferably, the synthetic organelle is constructed from biomacromolecular components selected from the group consisting of: nucleotide sequences with 10-1,000 consecutive repeats of repeat units, such as CAG triplets, CCUG, GGGAA, GGGGCC, etc.; amino acid sequences with 3-500 consecutive repeats, such as repeats of resilin-like peptides (RLP); intrinsically disordered regions, such as FUS protein, ParB protein; polymerization of multivalent biomacromolecules, such as PTB protein with UCUCU sequence repeats, artificially designed binary protein two-dimensional structures, and artificially designed binary RNA two-dimensional structures.

In one embodiment, the target product is one or more substances with a diameter in the range of 0.1-500 nanometers; preferably, the target product is produced biologically; and more preferably, the biological synthesis route of at least one component of the target product is encoded by a heterologous DNA, the component including: heterologously biosynthesized small molecules, such as deoxyviolacein; recombinantly expressed polypeptides, proteins, RNA, and DNA, such as green fluorescent protein, red fluorescent protein, T4 DNA ligase, 16S rRNA; and multi-component complexes, such as 30S ribosomal subunits, CRISPR-Cas9/gRNA complexes.

In one embodiment, step a comprises constructing a DNA construct, introducing the DNA construct into the host, and then guiding the formation of the synthetic organelle. The host comprises a cell-free expression system and a cell host, preferably a prokaryotic cell host, more preferably *E. coli*.

In one embodiment, step b comprises using the synthetic organelle to collect the target product. The collection method can be non-specific and/or specific binding, and the target product can originate outside the synthetic organelle and/or be generated within the synthetic organelle. This process can be achieved through non-specific diffusion and adsorption; or through specific intermolecular interactions by disposing a receptor domain in the synthetic organelle and attaching a corresponding ligand to the target product, such that the synthetic organelle having the receptor can specifically bind the target product having the ligand while preventing other impurities from entering the synthetic organelle. The target product may be produced in the synthetic organelle. For example, in one embodiment, deoxyviolacein is biosynthesized within the synthetic organelle. The target product may also be collected in the synthetic organelle from outside the organelle. For example, in one embodiment, MS2 capsid protein is fused to the N-terminus of recombinant green fluorescent protein, and MS2 hairpin RNA is connected to the 3' end of CAG repeats, in which case the synthetic organelle formed by the latter through liquid-liquid phase separation can specifically recruit GFP expressed in the cytosol. Preferably, the receptor/ligand pair includes: complementary nucleic acid molecule pairs; capsid proteins of RNA phages such as MS2, PP7, and Qbeta and translation operator RNA hairpins; protein N and boxB RNA hairpins of DNA phages such as λ, 21, and P22; CRISPR RNA hairpins and Cas6 protein of bacteria such as *Pseudomonas aeruginosa*; two segments of split proteins, such as split green fluorescent protein, alpha and omega fragments of beta-galactosidase, split T7 RNA polymerase; inducible dimerized or polymerized monomers, such as light-controlled polymerized green fluorescent protein Dronpa. Preferably, if in a host, the rate constant of target product influx into the synthetic organelle is higher than that of efflux, the target product will be enriched in the synthetic organelle, thereby allowing a higher yield of the target product. This property can be achieved by the physicochemical properties of the synthetic organelle and the target product. For example, the biosynthesized small molecule deoxyviolacein and various by-products produced in its biosynthetic pathway are poorly soluble in the aqueous phase, so they will be enriched in RNA condensates synthesized in the cell. It can also be achieved by high-affinity receptors and ligands.

In one embodiment, step c recovers the synthetic organelle containing the target product from the host. Preferably, step c comprises separation, recovery, and purification of the synthetic organelle, preferably comprising steps of:
c1. collecting and lysing the host;
c2. separating the pellet containing the synthetic organelle, preferably, the separation method is centrifugation and/or filtration or suction filtration;
c3. optionally, washing the pellet to remove impurities, thereby obtaining a purer synthetic organelle.

In one embodiment, step d releases the target product from the synthetic organelle containing the target product. Preferably, step d comprises separation, recovery, and purification of the target product, preferably comprising steps of:
d1. placing the synthetic organelle in an eluent. Due to the permeability of the synthetic organelle to target product, the target product will spontaneously diffuse into the eluent until an equilibrium is reached.

Preferably, if in the eluent, the rate constant of target product efflux out of the synthetic organelle is higher than that of influx, the synthetic organelle can be induced to accelerate the release of the target product, thereby allowing a higher yield of the target product. This property can be achieved by using an eluent with stronger affinity for the target product. For example, for deoxyviolacein, the eluent methanol has higher affinity for the target product than does the synthetic organelle RNA condensate. It can also be achieved by changing the affinity of the synthetic organelle, such as using a receptor and ligand pair sensitive to temperature, light signals, pH, etc. More preferably, the connection between the target product and the ligand can be broken, causing the synthetic organelle to lose specific binding to the target product, thereby enabling the synthetic organelle to efficiently release the target product without the ligand tag, for example, by using nickel ions, factor Xa, enterokinase, dithiothreitol (DTT), thrombin, or TEV protease to catalyze the cleavage of corresponding fusion linkers between proteins, by using imidazole to induce dCsy4 to cleave the 3' end of PA14 RNA hairpin, or by using ribozymes or inteins to catalyze self-cleavage, etc.
d2. separating the pellet containing the synthetic organelle to obtain the eluent containing the target product, preferably, the separation method is centrifugation and/or filtration or suction filtration;
d3. optionally, removing impurities from the eluent to obtain target substances with higher purity. Preferably, a separation and purification means is used to remove by-products from the eluent, such as using a molecular sieve to remove cleaved recombinant proteins, or using dialysis to remove metal ion contamination.

In another aspect, the present disclosure provides a DNA construct comprising, as defined in the above method, nucleotide sequence 1 that guides the host to construct the synthetic organelle, and nucleotide sequence 2 that guides and regulates the generation of the target product, preferably, wherein the DNA construct is composed of said nucleotide sequences 1 and 2 with a plasmid vector, and said plasmid vector is for example pACYC184, pACYC177, pET28a(+), pET28b(+), pET-5a(+), pET43.1a, pET-37b(+), pCDFDuet-1, pCOLADuet-1, pRSFDuet-1, pETDuet-1, pUC57, pUC19, pBAD, pBluescript II SK(+), pTrcHis C, pTrcHis A, pTrcHis2C, pBV221, pQE-70, pCold III, pRSET-CFP, pRSET-BFP, pGFPuv, pKD46, pKD4, pTYB1, PinPoint Xa-2, pTWIN1, pRSET C, pSB1C3, pSB3C5, pSB4C5, and pSB4K5.

In yet another aspect, the present disclosure provides an expression system comprising the above DNA construct. Preferably, the expression system refers to a system that simultaneously constructs the synthetic organelle and generates the target product in a prokaryotic cell, and more preferably, the expression system is an *E. coli* expression system.

### Beneficial Effects

The recovery and purification of a biologically synthesized target product require complex processes, generally involving the arrangement and combination of various unit operations such as extraction, chromatography, electrophoresis, centrifugation, and membrane treatment. These processes generally have defects such as cumbersome steps, long time consumption, and expensive equipment and consumables. Taking the purification of recombinant proteins as an example, chromatography is usually used, but has the following disadvantages: 1. Chromatographic columns and packing media are expensive; 2. Chromatographic columns have limited lifetime, and require cleaning and regeneration of the used medium before next use; 3. Different chromatographic methods need to be selected according to the properties of different molecules (including ion exchange chromatography, gel chromatography, size exclusion chromatography, hydrophobic chromatography, and affinity chromatography as main categories). These disadvantages limit the universality of chromatography methods and make it difficult to parallelize different processes on a large scale.

The method of the present disclosure has universality and can be used for recovery and purification of small molecules or ionic compounds, biomacromolecules, etc.; it is easy to operate, has simple processes, and is easy to scale up; it has low cost and generally does not require expensive equipment and/or consumables; it is highly standardized to an extent that basically the same procedure can be applied to different products, thereby enabling high-throughput parallel processing.

### Description of Drawings

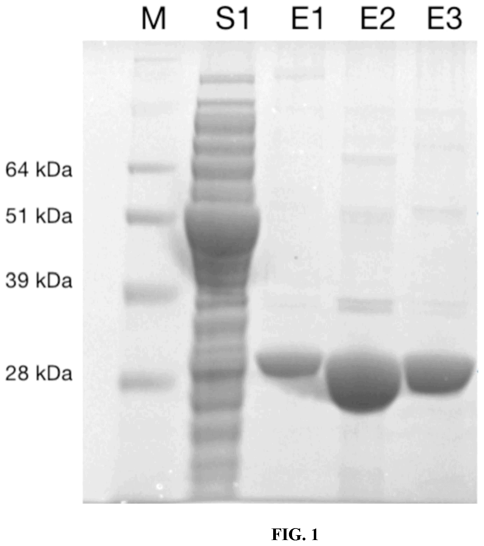

Figure 1 shows gel electrophoresis images for the target product sfGFP purified by the rCAG-MS2/tdMCP system. M: the pre-stained protein marker (GenScript, Catalog M00624); S1: whole-cell sample from the host induced at 37°C for 12 hours for expression, wherein the distinct band around 51 kDa corresponds to sfGFP fused with tdMCP-SNAC-tag; E1: product recovered from organelles 24 hours after nickel-ion cleavage of the SNAC-tag, wherein the distinct band around 28 kDa corresponds to the target product sfGFP; E2: product obtained 48 hours after the cleavage; E3: product obtained 72 hours after the cleavage.

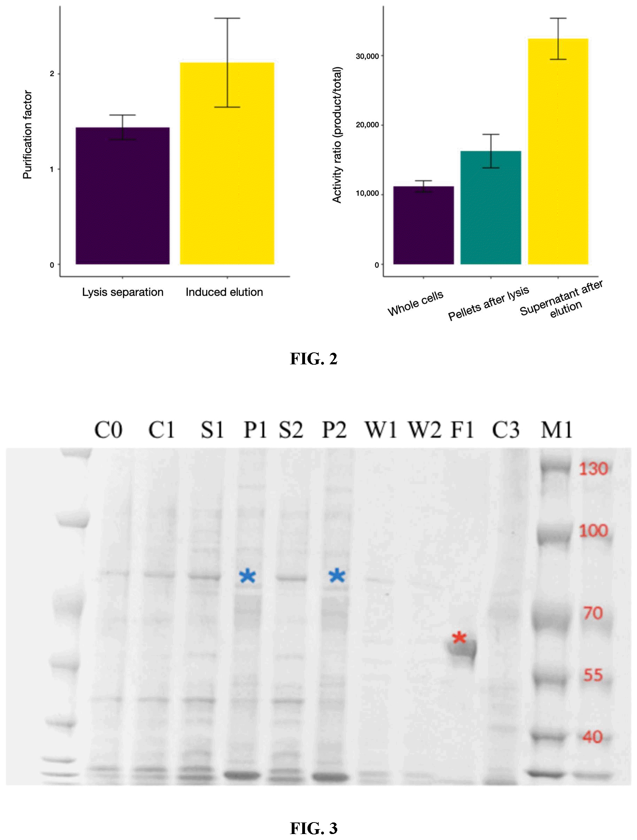

Figure 2 shows the relative quantitative analysis results obtained by using green fluorescent activity for the target product sfGFP purified by the rCAG-MS2/tdMCP system. The column chart on the left displays the purification efficiency at each step, wherein the "lysate separation" refers to step c, i.e. the purification step of recovering the target product from organelles after whole-cell lysis and deposition; and the "induced elution" refers to step d, i.e. the step of adding an inducer to the organelles to elute the target molecules. The right panel shows the relative activity of the target product to the total protein in different samples. Error bars are calculated standard deviations of triplet tests.
Figure 3 shows gel electrophoresis images for the target product T4 DNA ligase purified by the rCAG-MS2/tdMCP system. 1-Lane C0: uninduced whole cells; 2-Lane C1: whole cells induced at 37°C for 5 h; 3-Lane S1: supernatant from first cell lysate after induction at 37°C for 5 h; 4-Lane P1: pellet from first cell lysate after induction at 37°C for 5 h; 5-Lane S2: supernatant from second cell lysate after induction at 37°C for 5 h; 6-Lane P2: pellet from second cell lysate after induction at 37°C for 5 h; 7-Lane W1: buffer supernatant after washing the pellet; 8-Lane W2: buffer supernatant after second washing of the pellet; 9-Lane F1: supernatant from the cleaved product; 10-Lane C3: residual pellet after cleavage; Lane M1: pre-stained protein marker (GenScript, Catalog M00624).

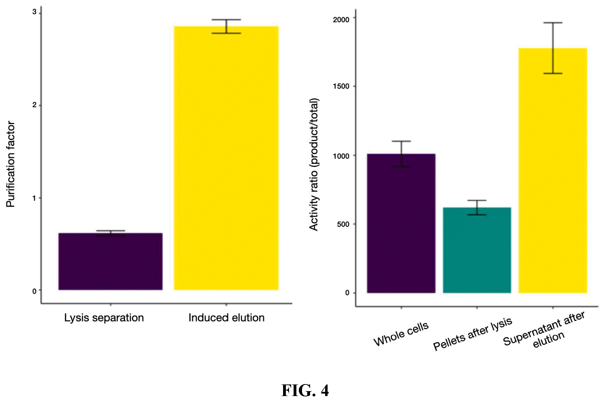

Figure 4 shows the relative quantitative analysis results obtained by using red fluorescence activity for the target product mKate2 purified by the RLP-GFP11/GFP(1-10) system. The column chart on the left displays the purification efficiency at each step, wherein the "lysate separation" refers to step c, i.e. the purification step of depositing the molecules enriched in organelles after whole-cell lysis; and the "induced elution" refers to step d, i.e. the step of adding an inducer to the organelles to elute the target molecules. The right panel shows the relative activity of the target product to the total protein in different samples. Error bars are calculated standard deviations of triplet tests.

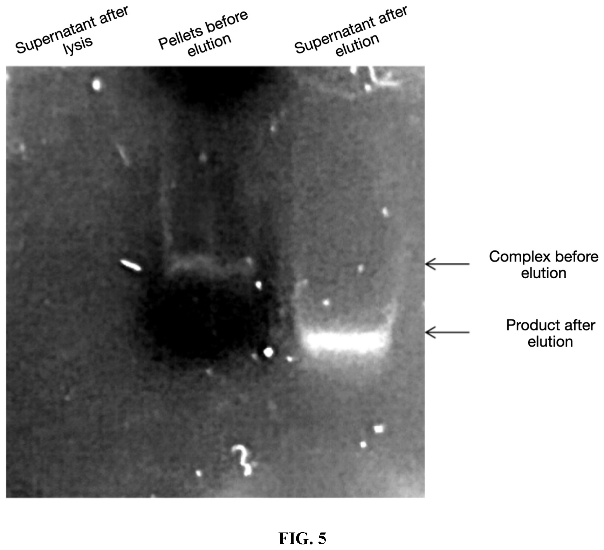

Figure 5 shows agarose gel electrophoresis images for the purification of the 30S ribosomal subunit comprising heterologously expressed 16S rRNA and endogenous ribosomal protein, by a system of a synthetic organelle constructed with ProteinB and ProteinA-dCsy4 and the PA14 CRISPR RNA hairpin as a ligand. The "supernatant after lysis" shows an extremely weak diffused band for RNA released from whole-cell lysate. The "pellets before elution" represents pellets of organelles after lysis, containing only a small amount of free ProteinA-dCsy4 complexes comprising RNA with the target product. The "supernatant after elution" contains the target product released after imidazole-induced dCsy4 cleavage of PA14 CRISPR RNA. The arrows on the right indicate molecular sizes before and after the cleavage.

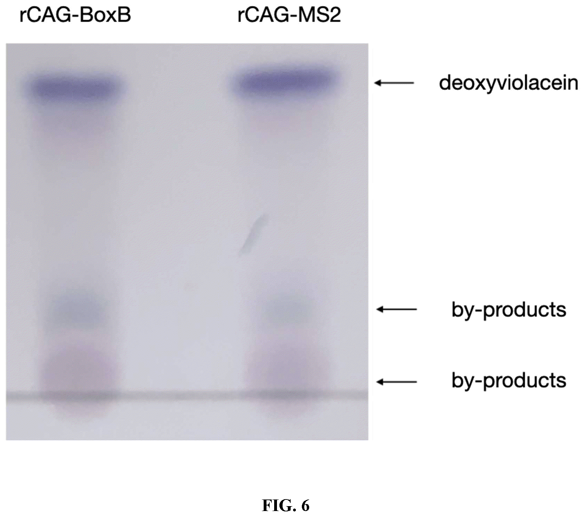

Figure 6 shows thin-layer chromatography results for the target product deoxyviolacein non-specifically recovered from the synthetic organelle constructed with rCAG-BoxB or rCAG-MS2. The sample on the left used a synthetic organelle constructed with rCAG-BoxB, wherein the target product was biosynthesized outside the organelle. The sample on the right used a synthetic organelle constructed with rCAG-MS2, wherein the target product was biosynthesized within the organelle. The arrows on the right indicate the eluted target product and by-product impurities.

### Detailed Description of Invention

The present disclosure is described in more detail below.
1. Synthetic Organelles

Cells generally have a complex spatial organization, one of which is called compartmentalization, referring to spatially organized structures formed within a cell to perform specific functions and biochemical reactions. Such compartmentalized structures are also often called organelles, which are to cells what organs are to individuals.

Organelles are either separately enclosed in their own lipid bilayers, called membrane-bound organelles, such as mitochondria, chloroplasts, etc.; or are spatially independent functional units without lipid bilayers around them, known as membraneless organelles, which are also compartments visible under optical microscopes. In 2009, Hyman and Brangwynne discovered and revealed that membraneless organelles are formed by biomacromolecules in cells through phase separation (1. C. P. Brangwynne et al., Germline P granules are liquid droplets that localize by controlled dissolution/condensation. Science 324, 1729-1732 (2009)).

Synthetic organelles are an innovative bioengineering concept, also called "organelle-like structures", "artificially synthesized organelles", or "artificial organelles". Synthetic organelles aim at designing and construction of small functional units similar to natural organelles for achieving compartmentalization in cells (or *in vitro* test tube environments), and are applied to control biosynthesis, metabolic pathways, or other biological processes. The construction of synthetic organelles involves introducing exogenous DNA constructs into host cells. These exogenous DNAs include exogenous coding genes, non-coding DNA, regulatory elements, etc., for guiding cell-free expression systems or host cells to synthesize specific polypeptides, proteins, DNA, RNA, enzymes, or other biomolecules. Some of these molecules serve to construct the synthetic organelle structure, while other molecules perform the preset functions of synthetic organelles, such as biocatalysis, receptor recognition, protein sorting, etc. Synthetic organelles can be constructed through heterologous multimer self-assembly of proteins, DNA, and RNA, or through modification of cell membrane structures. Since the phase separation mechanism for membraneless organelles was revealed, synthetic organelles can also be constructed by assembly of biomacromolecules capable of phase separation.

The present disclosure provides a method for recovering a target product by using a synthetic organelle. The synthetic organelle can be constructed substantially from polypeptides and/or proteins and/or DNAs and/or RNAs and/or nucleic acid-protein complexes. For example, repeated triplet ribonucleic acids (CAG)n spontaneously assemble into RNA condensates/droplets through liquid-liquid phase separation when transcribed in a cell. The synthetic organelle can also contain other substances according to the characteristics of a target product. For example, expressing P9 and P12 proteins of φ6 phage can recruit lipid molecules to form membrane structures.

The biomacromolecular components for constructing a synthetic membraneless organelle may be selected from the group consisting of: nucleotide sequences with 10-1,000 consecutive repeats of repeat units, such as CAG triplets, CCUG, GGGAA, GGGGCC, etc.; amino acid sequences with 3-500 consecutive repeats, such as repeats of resilin-like peptides (RLP); intrinsically disordered regions, such as FUS protein and ParB protein; polymerization of multivalent biomacromolecules, such as PTB protein with UCUCU sequence repeats; artificially designed binary protein two-dimensional structures; and artificially designed binary RNA two-dimensional structures.

Certainly, the synthetic organelle according to the present disclosure can also be constructed from polypeptides and/or proteins and/or DNAs and/or RNAs and/or nucleic acid-protein complexes of other sequences, for example:
· RNA:
   ∘ Repeated triplet ribonucleic acids, such as (CAG)n, (GGU)n, (AUG)n, (CGG)n, (GUU)n, (CGU)n, (AUU)n, (ACG)n, (AAU)n, (AUC)n, (CCG)n, (AGC)n, (AGU)n, (A CU)n, where n is 10-1,000;
   ∘ Repeated quadruplet ribonucleic acids, such as (CCUG)n, where n is 10-1,000;
   ∘ Repeated quintuplet ribonucleic acids, such as (GGGAA)n, where n is 10-1,000 ;
   ∘ Repeated sextuplet ribonucleic acids, such as GGGGCC(rGGGGCC), i.e., (GG GGCC)n, (AGGGCC)n, (GGAGCC)n, (GGGGAC)n, (AGGACC)n, where n is 10-1,000;
   ∘ Unary synthetic RNA scaffolds, for example **("XX--XX**" in bold represents sites where functional nucleic acid aptamers or ribozymes can be inserted): GGGTAGGCGC CTAGCCTAATGTACATTAAGTTATTTTTCCGGATGAATAGAATATATTCTAAT AACGCAGG**XX--XX**CCTCGAATACGAGCTGGG**XX--XX**CCCAGGAAGTGTTCG CACTTCTCTCGTATTCGATTGCGACTAGT;
   ∘ Binary synthetic RNA scaffolds, for example **("XX--XX**" in bold represents sites where functional nucleic acid aptamers or ribozymes can be inserted): d2-1 GGGTCAGGAATCCTCCTGATAGCTATTTGGACAATTACGTACGTAGTTGATGA CAACTACATGAAAATAAGGG**XX--XX**CCCTCTAGA and d2-2 GGGTAGTTGTTATGGATTCCTGATTTATGGG**XX--XX**CCACTAGT;
· DNA:
   ∘ ssDNA sequences identical to the above RNA sequences;
   ∘ ssDNA capable of forming nanopolymers, such as nanopolymers of the followi ng four ssDNAs **("XX--XX**" in bold represents sites where functional nucleic acid aptam ers or ribozymes can be inserted): oligo-1 TGCGCAATCCC**XX--XX**CTTGAGCACGC CAACATCACCGTAATT, oligo-2 GCGGCTAGCAGAGCATTCGGGA**XX--XX**GAC TATGGCTGTATTT, oligo-3 GGCGTGCTCAC**XX--XX**TCGGATTGCGCATGCTAG CCGCTATTT, oligo-4 CGGTGATGTTCAGCCATAGTAG**XX--XX**GCCGAATGCTC TATTT, etc.;
· Proteins:
   ∘ Self-assembled protein nanoparticles and nanostructures, such as: Carboxysome shell proteins; Metabolosome shell proteins; Bacterial microcompartment (BMC) shell proteins BMC-H, BMC-T, and BMC-P; *Haliangium ochraceum* bacterial microcompartment capsid protein T1 (HO BMC Shell T1); *Thermotoga maritima* encapsulation protein EncTm; Mixed phage Qβ virus-like particles; Ferritin; artificially synthesized protein nanocages, such as antibody Fc nanocages; artificially synthesized protein self-assembled fibers, such as CC-Di-B-PduA protein; artificially synthesized protein self-assembled two-dimensional structures; and artificially synthesized protein self-assembled three-dimensional structures, such as ATC-HL3 protein;
   ∘ Intrinsically disordered proteins (IDP), such as: Resilin-like peptides (RLP); Elastin-like peptides (ELP); RNA helicase LAF-1 RGG domain repeats; RNA helicase DDX4; *Caulobacter crescentus* ribonuclease E (RNaseE); IDP homologous protein sequences (GRGDSPYS)n, and mutants (GRGDSPYSGRGDSPYSGRGDSPYSGRGDSPVS)n, (GRGDSPYSGRGDSPVS)n, where n is 3-500; ParB protein; FUS protein; IbpA protein; PopZ protein; PodJ protein; conserved nuclear pole protein fiber protein FIB-1;
   ∘ Protein amyloid precipitates, such as: AEAEAKAKAEAEAKAK, LELELKLKLELELKLK, human β-amyloid peptide Ab42 (F19D);
   ∘ Other proteins that cause aggregation, such as: *E. coli* beta-galactosidase; Maltose-binding protein mutant MalE31; *Clostridium cellulovorans* cellulose-binding domain (CBD); *Clostridium thermocellum* endoglucanase D (EGD); fusion proteins of the following proteins from *Brucella abortus* and fluorescent proteins: PdhS-mCherry, FumC-YFP, and DivK-YFP; fusion proteins of the following proteins from *E. coli* and fluorescent proteins: ClpX-sfGFP, ClpP-sfGFP, ClpP-mCherry, ClpX-mCherry, ClpX-mCherry2, ClpX-Venus, LacZ-Dronpa, TetR-Dronpa, P22C2-Dronpa, HKCI-Dronpa; Tandem repeat SH3 (polySH3) with tandem repeat PRM (polyPRM); Tandem repeat SIM (polySIM) with tandem repeat SUMO (polySUMO); and Pentameric nuclear pole protein Npm1, etc.;
· Nucleoprotein multimer complexes:
   ∘ Complex of long non-coding RNA (lncRNA) and RNA-binding protein, wherein the nucleic acid scaffold include for example mammalian nuclear paraspeckle assembly transcript 1 isoform 2 in paraspeckles; intergenic spacer lncRNA in amyloid bodies; human satellite III (SatIII) lncRNA in nuclear stress bodies; Drosophila heat shock RNA (Hsr) ω; Schizosaccharomyces meiRNA; RNA-binding proteins such as PTB protein; FUS protein; and Histone BRD4;
   ∘ Polypeptide sequences and polynucleotides, such as polypeptide RRASLRRASL with polyuridylic acid (polyU) having a molecular weight of 600-1,000 kDa; synthetic polypeptides RP3, SR8, and polyU, etc.

Synthetic organelles may be constructed entirely from biomolecules synthesized by the host under the guidance of an exogenous DNA construct, may be constructed from partial exogenous molecules combined with endogenous molecules of the host, or may be obtained by modification of original endogenous organelles or cytoskeleton structures of the host. For example, in a method for codon expansion using synthetic organelles disclosed in EP 19157257.7, the synthetic organelle is formed by exogenous PylRS::FUS::KIF16B1-400 fusion protein, MCP::EWSR1::KIF16B1-400 fusion protein together with endogenous microtubule cytoskeleton structures.

It should be noted that although the synthetic organelles according to the present disclosure can be constructed in various ways and set no special requirements for the sources of their components, the synthetic organelles should be able to exist as a spatially independent structure, so that they are isolatable and recoverable. For example, in one embodiment, the host is *E. coli* cells, and the synthetic organelle is constructed from 46 repeats of triplet ribonucleic acid CAG, and is present in a pellet obtain after cell disruption and centrifugation, with impurities such as cytomatrix located in the supernatant. As a counterexample, if biomacromolecules form a specific spatial distribution in the host but fail to condense into an independent spatial structure, they cannot be separated from the cytomatrix. For example, the membraneless compartments disclosed in CN112575016A "Construction and Application of Membraneless Organelles in Prokaryotes" were in a soluble protein-enriched phase formed by recombinant spider fibroin or arthropod elastin-like protein, and after the host cell was disrupted, fusion proteins composed of spider fibroin or arthropod elastin-like protein and a target functional protein mainly existed in the supernatant after centrifugation, indicating that the membraneless compartments are not structures independent of the cytoplasmic matrix. Therefore, although they are also called "membraneless organelles", they cannot be separated and recovered, and thus do not fall within the scope of the synthetic organelles according to the present disclosure.

### 2. Collection of Target Products by Synthetic Organelles

Organelles generally have selective permeability and properties for sorting biomolecules. Therefore, the composition and physicochemical properties of biomolecules within the organelle compartments are significantly different from the components outside the organelles (such as the cytosol), thereby enabling organelles to perform specific functions. The present disclosure enables a synthetic organelle to collect a target product by designing the properties of the synthetic organelle and/or the target product.

In the present disclosure, collecting target products in the synthetic organelle can be achieved by non-specific diffusion and adsorption, or preferably by specific interactions between biomacromolecules, i.e., disposing a receptor domain in the constructed synthetic organelle and attaching a corresponding ligand to the target product. Since the ligand specifically targets the receptor, by this interaction the synthetic organelle can specifically bind the target product. The receptor and ligand can be selected as needed. For example, in one embodiment, the recruited ligand is tandem-dimer MS2 coat protein (tdMCP), and the receptor is MS2 hairpin RNA aptamer. In another embodiment, the ligand is protein N, and the receptor is BoxB hairpin RNA aptamer. Other ligand/receptor pairs may be used as needed, for example:
- Complementary nucleic acid molecules with any sequences;
- RNA-binding protein and corresponding RNA, for example:
   ∘ Capsid proteins of RNA phage and translation operator RNA hairpin, the RNA phage including MS2, PP7, Qbeta, etc.;
   ∘ Protein N and boxB RNA hairpin of DNA phage, the DNA phage including λ, 21, P22, etc.;
   ∘ CRISPR Cas protein and CRISPR RNA hairpin, for example Csy4 (Cas6f) derived from *Pseudomonas aeruginosa* PA14 strain and PA14 RNA hairpin;
   ∘ Other binding pairs, such as PUF-8 and 5'-UGUANAUA-3'; FBF-2 and 5'-UGURNNAUA-3'; protein A and protein A-binding aptamer; Archaeal RNA-binding protein L7Ae and RNA C/D box, etc.;
- DNA-binding protein and corresponding DNA, for example: TetR and tetO operator; cI repressor protein and cI operator; Gal4 and corresponding DNA; Zif268 DNA-binding domain and GATGCTGCA sequence; Gli-1 DNA-binding domain and GACCACCCAAGACGA sequence; LexA DNA-binding domain and CTGTATATATATACAG sequence; Transcription activator-like (TAL) domains and corresponding DNA;
- Homodimers or multimers, for example: reversible green fluorescent protein Dronpa;
- Split proteins, for example: split fluorescent proteins, such as split green fluorescent protein GFP1-10 and GFP11; split β-galactosidase; split T7 polymerase; split esterase; split TEV protease; split dihydrofolate reductase; split β-lactamase; split firefly luciferase; split thymidine kinase; split chorismate mutase; split CRISPR-Cas9; split horseradish peroxidase, etc.;
- Antigen and specific antibody, for example: His tag and anti-his antibody; flag tag and anti-flag antibody; HA tag and anti-HA antibody; Myc tag and anti-Myc antibody; GST protein and anti-GST antibody;
- Other protein-protein binding pairs, for example: SYNZIP peptide pair; SZ17 and SZ18 pair; Fos and Jun pair; *Arabidopsis thaliana* phytochrome B (PhyB) and PIF3 protein or PIF6 protein;

Certainly, receptor and ligand do not have to be a bimolecular pair, but may also form a complex multicomponent complex, such as the quaternary complex of CRISPR-Cas9 protein, crRNA, tracrRNA, and crRNA-targeted DNA; and the ternary complex of FKBP protein, FRB protein, and rapamycin.

In the synthetic organelles, receptors can be covalently connected to components that construct the synthetic organelle structure. For example, in one embodiment, the receptor MS2 hairpin RNA aptamer and the CAG repeat nucleotides that construct the synthetic organelle structure constitute a fusion RNA molecule. For example, in another embodiment, the receptor dCsy4 and protein A that constructs the synthetic organelle structure constitute a fusion protein molecule. Certainly, receptors can also be assembled in synthetic organelles through non-covalent intermolecular interactions. For example, CAG repeat nucleotides that construct the synthetic organelle structure are connected with MS2 hairpin RNA aptamer. The MS2 hairpin RNA aptamer non-covalently binds tdMCP-His tag. His tag non-covalently binds anti-His antibody and PhyB fusion protein. Then PhyB serves as a receptor, and the synthetic organelle can specifically bind proteins connected with lthe igand PIF3 or PIF6. The synthetic organelle can have one or more receptors with different properties.

The linkage between a ligand and a target product can be covalent. For example, in one embodiment, the ligand tdMCP and the target product green fluorescent protein constitute a fusion protein. For example, in another embodiment, the ligand CRISPR RNA hairpin structure and the target product 16S rRNA constitute a fusion RNA molecule. Certainly, ligands can also be attached to target products through non-covalent intermolecular interactions. For example, through intermolecular interactions, the target product Dronpa145N mutant can form a heterodimer with the ligand tdMCP-Dronpa145K. The latter can be specifically recruited by synthetic organelles with MS2 hairpin RNA. Then the synthetic organelle is separated and recovered, and Dronpa145N and tdMCP-Dronpa145K are dissociated by 500 nm light induction to obtain Dronpa145N. The target product can also be connected with one or more ligands.

In the present disclosure, during the collection process in step b, the target product can come from outside the synthetic organelle and then be collected by the synthetic organelle. For example, in one embodiment, heterologous green fluorescent protein GFP is expressed in the cytosol. The target product can also be directly generated in the synthetic organelle. For example, in one embodiment, the biosynthesized small molecule deoxyviolacein is directly biosynthesized in a synthetic organelle composed of RNA.

In the present disclosure, during the collection process in step b, the rate constant of target product influx into the synthetic organelle does not necessarily need to be higher than the rate constant of efflux out of the synthetic organelle. The target product will be partially distributed in the synthetic organelle, thereby allowing collection of the target product by the synthetic organelle. However, preferably, if the influx rate constant is higher than the efflux rate constant, the target product will be enriched in the synthetic organelle, thereby allowing a higher yield of the target product. The collection of the target product in the synthetic organelle can be achieved by utilizing physicochemical properties. For example, in one embodiment, the biosynthesized small molecule deoxyviolacein is poorly soluble in the aqueous phase and well soluble in the organic phase. Therefore, in cells, deoxyviolacein will be enriched in synthetic organelles composed of RNA. It can also be achieved by high-affinity receptor and ligand. For example, in one embodiment, the recruited ligand is a CRISPR RNA hairpin structure, and the receptor is the corresponding CRISPR Cas protein Csy4. Step b can be performed in a specific buffer, but more preferably, step b occurs in the host, and is performed simultaneously with the generation of the target product, to shorten the time required for the overall process and reduce operational complexity.

### 3. Release of Target Products by Synthetic Organelles

The present disclosure enables synthetic organelles to release a target product after mixing with an eluent by designing the properties of the synthetic organelles and/or the target product and/or the eluent.

In the present disclosure, in the eluent, the rate constant of target product efflux out of the synthetic organelle does not necessarily need to be higher than the influx rate constant. The target product will be partially distributed in the eluent, thereby allowing recovery of the target product. However, preferably, if in the eluent, the efflux rate constant is higher than the influx rate constant, the synthetic organelle can be induced to accelerate the release of the target product, thereby achieving a higher yield of the target product.

This property can be achieved by using an eluent with stronger affinity. For example, in one embodiment, deoxyviolacein has higher solubility in the eluent methanol than in the synthetic organelle, so that it can be released from the synthetic organelle.

This property can also be achieved by changing the affinity of the synthetic organelle for the target product. If the synthetic organelle uses a receptor to specifically recruit the target product connected with a ligand, then changing the affinity between the receptor and the ligand can accelerate the release of the target product. For example, receptor and ligand pairs sensitive to temperature, light signals, pH, etc. allow induction of the synthetic organelle to release the target product by the above signals.

More preferably, the connection between the target product and a ligand can be broken, causing the synthetic organelle to lose affinity for the target product, thereby inducing the synthetic organelle to efficiently release the target product without the ligand tag. For example, in one embodiment, the ligand protein binds to the target product protein via the SNAC-tag, and nickel ions are used to induce SNAC-tag cleavage so that the synthetic organelle releases the target product protein. For example, in another embodiment, the target product 16S rRNA and the ligand CRISPR repeat hairpin RNA form a fusion RNA molecule, and the ligand is bound by the receptor dCsy4. Imidazole is used to induce dCsy4 to cleave the 3' end of the CRISPR repeat hairpin RNA, so that the synthetic organelle releases the target product RNA. Disrupting the connection between the target product and a ligand can also be done in other ways, such as using factor Xa, enterokinase, dithiothreitol (DTT), thrombin, or TEV protease to cleave corresponding polypeptide sequences, or using ribozymes or inteins to catalyze self-cleavage, etc.

### 4. Target Products

The target product according to the present disclosure may be any molecule or structure intended to be prepared. The target product does not necessarily have to be a biologically synthesized molecule. For example, the target product may be heavy metal ions in industrial wastewater, or glucose absorbed by a host cell from the environment.

Preferably, the target product is a biologically synthesized molecule. For example, a biosynthesized small molecule may be microorganism-derived natural compounds such as violacein, deoxyviolacein, antibiotics, psilocybin; plant and animal-derived natural compounds such as carotenoids, indigo, artemisinin, 4-methyloctanoic acid, 4-methylnonanoic acid, C8-10 branched unsaturated fatty acids, carmine, paclitaxel; or recombinant polypeptides or proteins, such as superfolder green fluorescent protein (sfGFP), red fluorescent protein mKate2, T4 DNA ligase, artificially designed polypeptides; or recombinant RNA, such as *E. coli* 16S rRNA; or DNA, such as recombinant plasmids, single-stranded DNA; or complexes formed by recombinant macromolecules and endogenous macromolecules, such as 30S ribosomal subunits assembled from recombinantly expressed 16S rRNA and endogenous ribosomal proteins; or other cell structures different from the synthetic organelle, such as cytoskeleton and other organelles.

Preferably, the biosynthesis route of at least one component of the target product is encoded by a heterologous DNA, facilitating the regulation of the biosynthesis of the target product and connection of the target product with a suitable ligand to optimize the recovery efficiency of the target product.

The target product can be a mixture of multiple substances, such as a 1:1 mixture of green fluorescent protein and red fluorescent protein.

The target product can be synthesized biologically within the synthetic organelle, or synthesized biologically in the matrix outside the synthetic organelle.

### 5. DNA Constructs

According to the present disclosure, an artificially designed DNA construct is introduced into a host. The DNA construct is a polynucleotide containing multiple coding genes, non-coding DNA, regulatory elements, etc.

The DNA construct can guide the host to construct the synthetic organelle. For example, in one embodiment, the synthetic organelle is constructed from RNA molecules of CAG triplet nucleotide repeats, and the DNA construct contains a nucleotide sequence expressing this RNA molecule, including a sequence of CAG triplet nucleotide repeats, a transcription terminator, and a promoter regulating the inducible expression.

The DNA construct may also contain nucleotide sequences regulating the host to produce the target product. For example, in one embodiment, the DNA construct contains coding genes for four enzymes VioA, VioB, VioE, and VioC for synthesizing deoxyviolacein. For example, the DNA construct may contain an artificially synthesized RNA for regulating host metabolic pathways to increase the tryptophan yield in the host.

The DNA construct according to the present disclosure may be one or more plasmids, cosmids, or artificial chromosomes, may also be integrated into single or multiple sites in the host genome, or may be a combination of the above schemes. Preferably, the DNA construct is a plasmid composed of the nucleotide sequence guiding the host to construct the synthetic organelle and to produce the target product with a plasmid vector. The plasmid vector may be a commercially available vector, such as pACYC184, pACYC177, pET28a(+), pET28b(+), pET-5a(+), pET43.1a, pET-37b(+), pCDFDuet-1, pCOLADuet-1, pRSFDuet-1, pETDuet-1, pUC57, pUC19, pBAD, pBluescript II SK(+), pTrcHis C, pTrcHis A, pTrcHis2C, pBV221, pQE-70, pCold III, pRSET-CFP, pRSET-BFP, pGFPuv, pKD46, pKD4, pTYB1, PinPoint Xa-2, pTWIN1, or pRSET C, or modified structures of a commercially available vector, or non-commercial vectors, such as pSB1C3, pSB3C5, pSB4C5, and pSB4K5, or other artificially designed nucleotide sequences capable of stable replication in a host cell. For example, in one embodiment, the DNA construct is a dual-plasmid system, wherein one plasmid guides the host to generate a synthetic organelle constructed from RNA molecules of CAG triplet nucleotide repeats, with the vector being a variant of pACYC184, and the other plasmid guides the host to express recombinant protein sfGFP connected with the ligand tdMCP, with the vector being pET28a(+).

### 6. Expression Systems

The above DNA construct is introduced into a host for expression, guiding the host to construct the synthetic organelle, and/or regulating the host to produce the target product. The expression system may be a cell-free expression system and/or a cell expression system. The cell expression system includes prokaryotic cells and eukaryotic cells. Eukaryotic cells include, for example, yeast, fungal cells, animal cells, or plant cells, or prokaryotic cells include for example *E. coli*, *Bacillus subtilis*, lactic acid bacteria, bifidobacteria, and streptomycetes. Prokaryotic expression systems have the advantages of rapid cell reproduction, low culturing cost, and high yields, and are preferred, with *E. coli* being more preferred. For example, in one embodiment, the DNA construct is a triple-plasmid system, and the expression system is an *E. coli* expression system. The three plasmids are co-expressed in *E. coli*, with one guiding the host to construct the synthetic organelle, and the other two guiding the host to synthesize deoxyviolacein. The expression system may also involve a combined use of multiple systems. For example, a cell-free expression system is used to biosynthesize small-molecule compounds with high toxicity to cell growth, such as paclitaxel; an *E. coli* expression system is used to construct a synthetic organelle capable of recruiting paclitaxel; then the *E. coli* is lyzed and mixed with the former cell-free expression system, then the synthetic organelle is recovered, and finally paclitaxel is recovered from the synthetic organelle.

### 7. Target Product Recovery Method

The present disclosure provides a method for recovering a target product based on a synthetic organelle, comprising:
a. constructing a synthetic organelle in a host;
b. using the synthetic organelle to collect a target product;
c. recovering the synthetic organelle;
d. releasing the target product from the synthetic organelle, thereby obtaining the target product.

More specifically, step a refers to introducing a DNA construct into the host and guiding the host to construct the synthetic organelle; step b refers to using the synthetic organelle to collect the target product; step c comprises separation, recovery, and purification of the synthetic organelle; step d comprises releasing the target product from the synthetic organelle for separation, recovery, and purification of the target product.

Preferably, steps a and b are a process of simultaneously constructing the synthetic organelle, biologically producing the target product, and specifically enriching the target product in the synthetic organelle in a single host cell; step c is separation, recovery, and purification of the synthetic organelle; step d is separation, recovery, and purification of the target products, including:
c1. collecting and lysing the host;
c2. separating the pellet containing the synthetic organelle, preferably, the separation method is centrifugation and/or filtration;
c3. optionally, washing the pellet to remove impurities, thereby obtaining a purer synthetic organelle;
d1. placing the synthetic organelle in an eluent and inducing the synthetic organelle to release the target product, preferably, the induction method is a cleavage reaction, by using a catalyst to cleave the connection site between a ligand and the target substance, wherein the catalyst is for example nickel ions, imidazole-inducible dCsy4, factor Xa, enterokinase, dithiothreitol (DTT), thrombin, or TEV protease;
d2. separating the pellet containing the synthetic organelle to obtain the eluent containing the target product, preferably, the separation method is centrifugation and/or filtration;
d3. optionally, removing impurities from the eluent to obtain the target substance with higher purity.

### Examples

### Example 1: Purification of Fluorescent Protein through Organelle Synthesized by Phase Separation with Nucleic Acid Composed of CAG Repeats

### System Design

In this example, the molecules constituting the synthetic organelle were RNA fragments of 46 CAG repeats. This RNA can undergo liquid-liquid phase separation through interactions. This example used tandem-dimer MS2 coat protein (tdMCP) as the ligand and MS2 RNA hairpin as the receptor to enrich superfolder green fluorescent protein (sfGFP). SNAC-tag, which undergoes self-cleavage induced by divalent nickel ions, was used as the protein fusion linker and cleavage site between the tdMCP and sfGFP. Two plasmids pGFP and prCAG-MS2 were used in this example, expressing tdMCP-SNAC-sfGFP fusion protein and rCAG-MS2 RNA, respectively. The experiment was performed according to the following process.

### Experimental Process

pGFP and prCAG-MS2 were co-transformed into *E. coli* BL21(DE3) competent cells (TransGen). Single colonies were picked and cultured under shaking at 37°C, 220 rpm for 16 hours. To the flask 0.5 mM isopropyl β-D-1-thiogalactopyranoside (IPTG) (Sangon Biotech) and 150 ng/ml anhydrotetracycline hydrochloride (aTc) (J&K Scientific) were added as inducers to induce expression during culturing overnight for about 12 hours. The bacterial culture was centrifuged and the supernatant was discarded. A lysis buffer (One-Step Bacterial Lysis Kit, Sangon Biotech) was added to the pellet, followed by mixing by pipetting, a reaction at room temperature for 30 min, and then centrifuging. The supernatant was discarded and the pellet was recovered. A cleavage eluent containing nickel ions was added to the pellet obtained in the previous step to allow cleavage at room temperature on a rotary mixer . The sample after the cleavage reaction was centrifuged, and the supernatant was recovered, which was a solution containing the target product protein.

Subsequently, SDS-PAGE electrophoresis was conducted to test the protein samples (SurePAGE^{™}, GenScript) for the size, yield, and purity of the recovered protein.

Total protein/corresponding fluorescence was measured for each of the whole cell/pellet after first lysis/supernatant after elution. After subtracting the background from all the data, three indicators were calculated: specific activity (SA), purification factor (PF), and yield. Specific activity (SA) is the ratio of the target product to total protein in the sample after each step (target product / total protein). Purification factor (PF) is the ratio of specific activity after a step to specific activity before that step, used to characterize the efficiency at each step. Yield is calculated as the ratio of the total amount of the target product in the sample after each step to the total amount thereof before purification, used to evaluate the final purification yield.

### Results

Results are shown in Figures 1 and 2. The gel image shows that the finally eluted sfGFP had high purity with no obvious impurity bands. A semi-quantitative analysis shows that the product purification factor (PF) after the step of recovering the synthetic organelle was about 1.5 folds, and the product purification factor (PF) after the step of recovering the target product was about 2 folds. The specific activity (SA) of products was significantly improved in both steps, proving that the system combining the synthetic organelle and the corresponding process in this example can be used for product purification.

### Sequence Information

The size of sfGFP is 26 kDa, and the protein sequence thereof is shown in SEQ ID NO:1 below:

MSKGEELFTGVVPILVELDGDVNGHKFSVRGEGEGDATNGKLTLKFICTT
GKLPVPWPTLVTTLTYGVQCFSRYPDHMKRHDFFKSAMPEGYVQERTISFKDDG
TYKTRAEVKFEGDTLVNRIELKGIDFKEDGNILGHKLEYN FNSHNVYITADKQK
NGIKANFKIRHNVEDGSVQLADHYQQNTPIGDGPVLLPDNHYLSTQSVLSKDPN
EKRDHMVLLEFVTAAGITHG (SEQ ID NO:1).

The RNA sequence of rCAG-MS2 is shown in SEQ ID NO:2 below:

cagcagcagcagcagcagcagcagcagcagcagcagcagcagcagcagcagcagcagcagcagcagcagcagcagca
gcagcagcagcagcagcagcagcagcagcagcagcagcagcagcagcagcagcagcagcagcagcagagacggagcu
cgucgacgcggccgcaagcuugguaccgagcucggauccuaagguaccuaauugccuagaaaacaugaggaucacc
caugucugcaggucgacucuagaaaacaugaggaucacccaugucugcaguauucccggguucauuagauccuaag
guaccuaauugccuagaaaacaugaggaucacccaugucugcaggucgacucuagaaaacaugaggaucacccaug
ucugcaguauucccggguucauuagauccuaagguaccuaauugccuagaaaacaugaggaucacccaugucugca
ggucgacucuagaaaacaugaggaucacccaugucugcaguauucccggguucauuagauccuaagguaccuaauu
gccuagaaaacaugaggaucacccaugucugcaggucgacucuagaaaacaugaggaucacccaugucugcaguau
ucccggguucauuagauccuaagguaccuaauugccuagaaaacaugaggaucacccaugucugcaggucgacucu
agaaaacaugaggaucacccaugucugcaguauucccggguucauuagauccuaagguaccuaauugccuagaaaa
caugaggaucacccaugucugcaggucgacucuagaaaacaugaggaucacccaugu (SEQ ID NO:2)

The protein sequence of tdMCP is shown in SEQ ID NO:3 below:

MASNFTQFVLVDNGGTGDVTVAPSNFANGVAEWISSNSRSQAYKVTCSVRQSS
AQNRKYTIKVEVPKVATQTVGGVELPVAAWRSYLNMELTIPIFATNSDCELIVKA
MQGLLKDGNPIPSAIAANSGIYANFTQFVLVDNGGTGDVTVAPSNFANGVAEWI
SSNSRSQAYKVTCSVRQSSAQNRKYTIKVEVPKVATQTVGGVELPVAAWRSYL
NMELTIPIFATNSDCELIVKAMQGLLKDGNPIPSAIAANSGIY (SEQ ID NO:3)

The protein sequence of SNAC-tag is shown in SEQ ID NO:4 below:
GSHHW (SEQ ID NO:4)

### Example 2: Purification of Enzyme through Organelle Synthesized by Phase Separation with Nucleic Acid Composed of CAG Repeats

### System Design

In this example, the molecules constituting the synthetic organelle, the receptor, and the ligand, and the mechanism for cleaving and releasing the product, were all the same as in Example 1, except that the target product was T4 DNA ligase. Two plasmids pT4 and prCAG-MS2 were used in this example, expressing tdMCP-SNAC-T4 DNA ligase fusion protein and rCAG-MS2 RNA, respectively. The experiment was performed according to the following process.

### Experimental Process

The pT4 and prCAG-MS2 were co-transformed into E. coli BL21(DE3) competent cells (TransGen). Single colonies were picked and cultured under shaking at 37°C, 220 rpm for 16 hours. To the flask 0.5 mM IPTG (Sangon Biotech) and 150 ng/ml aTc (J&K Scientific) were added as inducers to induce expression during culturing for about 5 hours. The bacterial culture was centrifuged and the supernatant was discarded. A lysis buffer (One-Step Bacterial Lysis Kit, Sangon Biotech) was added to the pellet, followed by mixing by pipetting, a reaction at room temperature for 30 min (the first lysis), and then centrifuging to separate supernatant and pellet. A lysis buffer was added to the pellet obtained in the previous step again, followed by mixing by pipetting, a reaction at room temperature for 30 min (the second lysis), and then centrifuging to separate supernatant and pellet. A lysis buffer diluted 10-fold was added to the pellet obtained in the previous step, followed by mixing by pipetting, and then centrifuging to separate supernatant and pellet (the first wash). A lysis buffer diluted 10-fold was added to the pellet obtained in the previous step again, followed by mixing by pipetting, and then centrifuging to separate supernatant and pellet (the second wash). The pellet obtained in the previous step was mixed with a cleavage eluent containing nickel ions and placed on a rotary mixer to carry out room temperature cleavage. The sample after the cleavage reaction was centrifuged, and the supernatant was recovered, which was a solution containing the target product protein. Subsequently, SDS-PAGE electrophoresis was conducted to test the protein sample (SurePAGE^{™}, GenScript) for the size, yield, and purity of the recovered protein.

### Results

Results are shown in Figure 3. The gel image shows that the finally eluted T4 DNA ligase had high purity with no obvious impurity bands. Multiple washes can remove a small amount of impurities.

### Sequence Information

The size of T4 DNA ligase is 55.3 kDa, and its protein sequence is shown in SEQ ID NO:5 below:

MILKILNEIASIGSTKQKQAILEKNKDNELLKRVYRLTYSRGLQYYIKKWPKPGIA
TQSFGMLTLTDMLDFIEFTLATRKLTGNAAIEELTGYITDGKKDDVEVLRRVMM
RDLECGASVSIANKVWPGLIPEQPQMLASSYDEKGINKNIKFPAFAQLKADGARC
FAEVRGDELDDVRLLSRAGNEYLGLDLLKEELIKMTAEARQIHPEGVLIDGELVY
HEQVKKEPEGLDFLFDAYPENSKAKEFAEVAESRTASNGIANKSLKGTISEKEAQ
CMKFQVWDYVPLVEIYSLPAFRLKYDVRFSKLEQMTSGYDKVILIENQVVNNLD
EAKVIYKKYIDQGLEGIILKNIDGLWENARSKNLYKFKEVIDVDLKIVGIYPHRKD
PTKAGGFILESECGKIKVNAGSGLKDKAGVKSHELDRTRIMENQNYYIGKILECE
CNGWLKSDGRTDYVKLFLPIAIRLREDKTKANTFEDVFGDFHEVTGL (SEQ ID
NO:5)

### Example 3: Purification of Protein through Organelle Synthesized by Phase Separation with Protein Composed of RLP Repeats

### System Design

In this example, the molecules constituting the synthetic organelle were polypeptide fragments of 20 repeats of resilin-like peptides (RLP). This protein can undergo liquid-liquid phase separation through interactions. This example used the two parts of split green fluorescent protein (split GFP), GFP1-10 and GFP11, as the ligand and receptor, respectively, to enrich the target product red fluorescent protein mKate2. SNAC-tag was used to connect GFP1-10 and mKate2. Two plasmids, pET28a-RLP-sfGFP(1-10) and pCDF-sfGFP11-SNAC-mKate2, were used in this example, expressing 20xRLP-7xGFP11 fusion protein and GFP(1-10)-SNAC-mKate2 fusion protein, respectively.

### Experimental Procedure

The two plasmids pET28a-RLP-sfGFP(1-10) and pCDF-sfGFP11-SNAC-mKate2 were co-transformed into *E. coli* BL21(DE3) competent cells (TransGen). Single colonies were picked, and the bacterial solution was cultured at 37°C, 220 rpm for 16 hours. To the flask 0.5 mM IPTG (Sangon Biotech) and 150 ng/ml aTc (J&K Scientific) were added as inducers, followed by culturing overnight for about 12 hours. The bacterial culture was centrifuged and the supernatant was discarded. A lysis buffer (One-Step Bacterial Lysis Kit, Sangon Biotech) was added to the pellet, followed by mixing by pipetting, and a reaction at room temperature for 30 min. The pellet obtained in the previous step was mixed with a cleavage buffer containing nickel ions and placed on a rotary mixer to carry out room temperature cleavage. The sample after the cleavage reaction was centrifuged, and the supernatant was recovered, which was a solution containing the target product protein.

Subsequently, total protein/corresponding fluorescence was measured for each of the whole cell/pellet after first lysis/supernatant after elution. After subtracting the background from all the data, three indicators were calculated: specific activity, purification factor, and yield. Specific activity is the ratio of the target product to total protein in the sample after each step. Purification factor is the ratio of specific activity after a step to specific activity before that step, used to characterize the efficiency at each step. Yield is calculated as the ratio of the total amount of the target product in the sample after each step to the total amount thereof before purification, used to evaluate the final purification yield.

### Results Analysis

The detailed results are shown in Figure 4. A semi-quantitative analysis showed that over the two steps, the purification factor of the mKate2 product reached 1.76, and the specific activity of the product was significantly different from that before purification, proving that when the synthetic organelle is synthesized with proteins and interacting proteins are used as receptor/ligand, this system and process can be used for product recovery and purification. In particular, the specific activity of the product after the step of recovering the synthetic organelle decreased, and the product purification factor was about 0.6 folds, indicating that the proportion of mKate2 in the synthetic organelle was actually significantly smaller than its proportion in the entire cell. Therefore, this result demonstrates that in the present disclosure, even if the affinity of the synthetic organelle in step b is insufficient to achieve enrichment of the target product in the synthetic organelle relative to the cytosol outside the organelle, it can still be used for recovery and purification of the target product.

### Sequence Information

The 7xGFP11 protein sequence is shown in SEQ ID NO:6 below:

RDHMVLHEYVNAAGITGGSGGRDHMVLHEYVNAAGITGGSGGRDHMVLHEYV
NAAGITGGSGGRDHMVLHEYVNAAGITGGSGGRDHMVLHEYVNAAGITGGSG
GRDHMVLHEYVNAAGITGGSGGRDHMVLHEYVNAAGIT (SEQ ID NO:6)

The GFP1-10 protein sequence is shown in SEQ ID NO:7 below:

MSKGEELFTGVVPILVELDGDVNGHKFSVRGEGEGDATIGKLTLKFICTTGKLPV
PWPTLVTTLTYGVQCFSRYPDHMKRHDFFKSAMPEGYVQERTISFKDDGKYKTR

AVVKFEGDTLVNRIELKGTDFKEDGNILGHKLEYNFNSHNVYITADKQKNGIKA
NFTVRHNVEDGSVQLADHYQQNTPIGDGPVLLPDNHYLSTQTVLSKDPNEK
(SEQ ID NO:7)

The mKate2 protein sequence is shown in SEQ ID NO:8 below:

MSELIKENMHMKLYMEGTVNNHHFKCTSEGEGKPYEGTQTMRIKAVEGGPLPF
AFDILATSFMYGSKTFINHTQGIPDFFKQSFPEGFTWERVTTYEDGGVLTATQDTS
LQDGCLIYNVKIRGVNFPSNGPVMQKKTLGWEASTETLYPADGGLEGRADMAL
KLVGGGHLICNLKTTYRSKKPAKNLKMPGVYYVDRRLERIKEADKETYVEQHE
VAVARYCDLPSKLGHR (SEQ ID NO:8)

The 20-repeat RLP has a size of 17 kDa, and the protein sequence is shown in SEQ ID NO:9 below:

SKGPGRGDSPYSGRGDSPYSGRGDSPYSGRGDSPYSGRGDSPYSGRGDSPYSGR
GDSPYSGRGDSPYSGRGDSPYSGRGDSPYSGRGDSPYSGRGDSPYSGRGDSPYSG
RGDSPYSGRGDSPYSGRGDSPYSGRGDSPYSGRGDSPYSGRGDSPYSGY (SEQ ID
NO:9)

### Example 4: Purification of RNA through Synthetic Organelle Composed of Artificially Designed Binary Protein Two-Dimensional Structure

### System Design

In this example, the molecules constituting the synthetic organelle were two artificially designed proteins, protein A and protein B. Proteins A/B can self-assemble in a cell into a two-dimensional planar nanostructure. The receptor used in this example was a conditionally inactivated mutant dCsy4 of CRISPR Cas protein Csy4 from *Pseudomonas aeruginosa* PA14 strain. The ligand was the corresponding CRISPR RNA repeat hairpin sequence (PA14 RNA). The target product was a 30S ribosomal subunit assembled from recombinantly expressed 16S rRNA from *E. coli* and endogenous ribosomal protein. PA14 RNA and 16S rRNA were directly connected to form a fusion RNA molecule. The dCsy4 protein fused to protein A can specifically recognize and bind PA14 RNA, and cleave the 3' end of PA14 RNA under imidazole induction to release the target product. Two plasmids were used in this example, expressing ProteinB, ProteinA-dCsy4 fusion protein, and PA14-16S rRNA, respectively. The experiment was performed according to the following process.

### Experimental Process

The two plasmids were co-transformed into *E. coli* BL21(DE3) competent cells (TransGen). Single colonies were picked, and the bacterial solution was cultured at 37°C, 220 rpm for 16 hours. To the flask to 0.5 mM IPTG (Sangon Biotech) and 150 ng/ml aTc (J&K Scientific) were added as inducers, followed by culturing overnight. The bacterial culture was centrifuged and the supernatant was discarded. A lysis buffer (One-Step Bacterial Lysis Kit, Sangon Biotech) was added to the pellet, followed by mixing by pipetting, and a reaction at room temperature for 30 min. The pellet obtained in the previous step was mixed with an eluent containing 300 mM imidazole and placed on a rotary mixer to carry out room temperature elution. The sample after the cleavage reaction was centrifuged, and the supernatant after elution was obtained, which was a solution containing the target product protein. Agarose gel electrophoresis was performed for each of the whole cell sample/pellet after first lysis/supernatant after elution.

### Results

The detailed results are shown in Figure 5. In this example, there was a clear and bright band in the eluted product with no obvious impurity bands. The results prove that this design and experimental framework can be used for recovery and purification of RNA and its complexes. There was an obviously migrated band in the pellet recovered before elution, but the signal was very weak, indicating that before the cleavage reaction, there were a small amount of complexes of free target product from the synthetic organelle bound with ProteinA-dCsy4 fusion protein.

### Sequence Information

The RNA sequence corresponding to 16S rRNA template is shown in SEQ ID NO:10 below:

aguuugaucauggcucagauugaacgcuggcggcaggccuaacacaugcaagucgaacgguaacaggaagcagcuu
gcugcuuugcugacgaguggcggacgggugaguaaugucugggaaacugccugauggagggggauaacuacugga
aacgguagcuaauaccgcauaacgucgcaagcacaaagagggggaccuuagggccucuugccaucggaugugccca
gaugggauuagcuaguaggugggguaacggcucaccuaggcgacgaucccuagcuggucugagaggauaccagca
acacuggaacugagacacgguccagacuccuacgggaggcagcaguggggaauauugcacaaugggcgcaagccug
augcagccaugcngcguguaugaagaaggccuucggguuguaaaguacuuucagcggggaggaagggaguaaagu
uaauaccuuugcucauugacguuacccgcagaagaagcaccggcuaacuccgugccagcagccgcgguaauacgga
gggugcaagcguuaaucggaauuacugggcguaaagcgcacgcaggcgguuuguuaagucagaugugaaaucccc
gggcucaaccugggaacugcaucugauacuggcaagcuugagucucguagagggggguagaauuccagguguagc
ggugaaaugcguagagaucuggaggaauaccgguggcgaaggcggcccccuggacgaagacugacgcucaggugc
gaaagcguggggagcaaacaggauuagauacccugguaguccacgccguaaacgaugucgacuuggagguugugcc
cuugaggcguggcuuccggannuaacgcguuaagucgaccgccuggggaguacggccgcaagguuaaaacucaaau
gaauugacgggggccgcacaagcgguggagcaugugguuuaauucgaugcaacgcgaagaaccuuaccuggucuu

gacauccacggaaguuuucagagaugagaaugugccuucgggaaccgugagacaggugcugcauggcugucguca
gcucguguugugaaauguuggguuaagucccgcaacgagcgcaacccuuauccuuuguugccagcgguccggccg
ggaacucaaaggagacugccagugauaaacuggaggaagguggggaugacgucaagucaucauggcccuuacgacc
agggcuacacacgugcuacaauggcgcauacaaagagaagcgaccucgcgagagcaagcggaccucauaaagugcgu
cguaguccggauuggagucugcaacucgacuccaugaagucggaaucgcuaguaaucguggaucagaaugccacgg
ugaauacguucccgggccuuguacacaccgcccgucacaccaugggaguggguugcaaaagaaguagguagcuuaa
cuucgggagggcg (SEQ ID NO:10)

The RNA sequence corresponding to PA14 is shown in SEQ ID NO:11 below:
guucacugccguauaggcagcuaagaaa (SEQ ID NO:11)

The protein sequence corresponding to dCsy4 is shown in SEQ ID NO:12 below:

MDHYLDIRLRPDPEFPPAQLMSVLFGKLAQALVAQGGDRIGVSFPDLDESRSRLG
ERLRIHASADDLRALLARPWLEGLRDHLQFGEPAVVPHPTPYRQVSRVQAKSNP
ERLRRRLMRRHDLSEEEARKRIPDTVARALDLPFVTLRSQSTGQHFRLFIRHGPL
QVTAEEGGFTCYGLSKGGFVPWF (SEQ ID NO:12)

The protein sequence corresponding to ProteinB is shown in SEQ ID NO:13 below:

MGSLITLVELEWLEHQLIVQLSERLKGQIAKVGELLCECLKKGGKILICGNGGSA
ADAQHFAAELSGRYKKERKALAGIALTTDTSALSAIGNDYGFEFVFSRQVEALG
NEKDVLIGISTSGKSPNVLEALKKAKELNMLCLGLSGKGGGMMNKLCDHNLVV
PSDTARIQEMHILIIHTLCQIIDESFLEHHHHHH (SEQ ID NO:13)

The protein sequence corresponding to ProteinA is shown in SEQ ID NO:14 below:

MGHHHHHHGGLALVATGNDTTTKPDLYYLKNSEAINSLALLPPPPAVGSIAFLN
DQAMYEQGRLLRNTERGKLAAEDANLSSGGVANAFSGAFGSPITEKDAPALHKL
LTNMIEDAGDLATRSAKDHYMRIRPFAFYGVSTCNTTEQDKLSKNGSYPSGHTSI
GWATALVLAEINPQRQNEILKRGYELGQSRVICGYHWQSDVDAARVVGSAVVA
TLHTNPEFQAQLIKAKIEFKQHQKEL (SEQ ID NO:14)

### Example 5: Recovery of Deoxyviolacein through Organelle Synthesized by Phase Separation with Nucleic Acid Composed of CAG Repeats

### System Design

In this example, the target product was deoxyviolacein (DV). Purple deoxyviolacein having antibacterial and anticancer effects was converted from L-tryptophan sequentially catalyzed by four enzymes VioA, VioB, VioE, and VioC derived from *Chromobacterium violaceum*, with an absorption peak around 570 nm. In this example, for strain 1, three plasmids were co-expressed in the host, wherein prCAG-MS2 was used to construct a synthetic organelle composed of CAG repeats and MS2 RNA hairpin repeats, and pVioAMBMEM and pVioCM were co-expressed, expressing VioA, VioB, VioE, and VioC enzymes each having tdMCP connected at the C-terminus. For Strain 2, three plasmids were co-expressed in the host, wherein prCAG-BoxB was used to construct a synthetic organelle composed of CAG repeats and BoxB RNA hairpin repeats, and pVioAMBMEM and pVioCM were co-expressed, expressing the above four fusion proteins of tdMCP and enzymes. The sequences of pVioAMBMEM and pVioCM plasmids are all from Guo, Haotian, et al. "Spatial engineering of E. coli with addressable phase-separated RNAs." Cell 185.20 (2022):3823-3837. Due to the high specificity of the BoxB and MS2 system, in strain 1 and strain 2, the enzymes VioA, VioB, VioE, and VioC will be localized inside and outside the synthetic organelle, respectively, such that the generated DV is located in the synthetic organelle and in the cytosol, respectively. In this example, the collection of DV by the synthetic organelle was not by specific receptor/ligand binding, but DV can only be distributed inside and outside the synthetic organelle through diffusion and non-specific dissolution and adsorption in the host cell.

### Experimental Process

The specified plasmid sets were co-transformed into *E. coli* BL21AI competent cells to obtain strain 1 and strain 2. Single colonies were picked, cultured under shaking at 37°C, 150 rpm for 16 hours, then diluted 1:200 in a 5 mL culture medium, and cultured at 37°C, 150 rpm for 4 hours to the logarithmic phase. aTc was added to a final concentration of 50 ng/mL, following by culturing for another 20 hours. The bacterial culture was centrifuged and the supernatant was discarded to obtain a pellet containing cells. The cells were re-suspended with phosphate buffered saline (PBS) and centrifuged to wash the cells, repeated twice. The cells were re-suspended with PBS, and a lysis buffer (Bugbuster, Sigma-Aldrich) was added, followed by mixing by pipetting, a reaction under shaking at room temperature at 900 rpm for 20 min, and then centrifuging. The supernatant was discarded, and the pellet was recovered. Methanol was added, followed by shaking for 30 seconds and centrifuging. The supernatant was the eluent containing the target product. 200 µL of the eluent was taken and subjected to thin-layer chromatography analysis on a silica gel thin-layer chromatography plate (TLC Silica gel 60, Merck, Germany) using a 6:1 chloroform-methanol solution.

### Results

Results are shown in Figure 6. The chromatography developed color under visible light, indicating that the methanol elution recovered the target product DV, along with a small amount of other by-product impurities, as marked by arrows. The recovery rate of the target product was similar between the two strains. This proves that this design and experimental framework can be used for recovery of small molecule compounds; and proves that this design and experimental framework are insensitive to the synthesis location of the target product, which can be either inside or outside the organelle.

### Sequence Information

The RNA sequence corresponding to rCAG-BoxB is shown in SEQ ID NO:15 below:

cagcagcagcagcagcagcagcagcagcagcagcagcagcagcagcagcagcagcagcagcagcagcagca
gcagcagcagcagcagcagcagcagcagcagcagcagcagcagcagcagcagcagcagcagagacggagctcgtc
gacgcggccaagcttggtaccgagctcgggccctgaagaagggcccctcgactaagtccaactactaaactgggccctgaag
aagggcccatatagggccctgaagaagggcccctcgactaagtccaactactaaactgggccctgaagaagggcccatatag
ggccctgaagaagggcccctcgactaagtccaactactaaactgggccctgaagaagggcccatatagatcaagcttggtacc
gagctcgggccctgaagaagggcccctcgactaagtccactactaaactgggccctgaagaagggcccatatagggccctga
agaagggcccctcgactaagtccaactactaaactgggccctgaagaagggcccatatagggccctgaagaagggcccctcg
actaagtccaactactaaactgggccctgaagaagggcccatatatcgagtaaggatct (SEQ ID NO:15)

The amino acid sequence corresponding to VioA is shown in SEQ ID NO:16 below:

MKHSSDICIVGAGISGLTCASHLLDSPACRGLSLRIFDMQQEAGGRIRSKMLDGK
ASIELGAGRYSPQLHPHFQSAMQHYSQKSEVYPFTQLKFKSHVQQKLKRAMNEL
SPRLKEHGKESFLQFVSRYQGHDSAVGMIRSMGYDALFLPDISAEMAYDIVGKH
PEIQSVTDNDANQWFAAETGFAGLIQGIKAKVKAAGARFSLGYRLLSVRTDGDG
YLLQLAGDDGWKLEHRTRHLILAIPPSAMAGLNVDFPEAWSGARYGSLPLFKGF
LTYGEPWWLDYKLDDQVLIVDNPLRKIYFKGDKYLFFYTDSEMANYWRGCVAE
GEDGYLEQIRTHLASALGIVRERIPQPLAHVHKYWAHGVEFCRDSDIDHPSALSH
RDSGIIACSDAYTEHCGWMEGGLLSAREASRLLLQRIAA (SEQ ID NO:16)

The amino acid sequence corresponding to VioB is shown in SEQ ID NO:17 below:

MSILDFPRIHFRGWARVNAPTANRDPHGHIDMASNTVAMAGEPFDLARHPTEFH
RHLRSLGPRFGLDGRADPEGPFSLAEGYNAAGNNHFSWESATVSHVQWDGGEA
DRGDGLVGARLALWGHYNDYLRTTFNRARWVDSDPTRRDAAQIYAGQFTISPA

GAGPGTPWLFTADIDDSHGARWTRGGHIAERGGHFLDEEFGLARLFQFSVPKDH
PHFLFHPGPFDSEAWRRLQLALEDDDVLGLTVQYALFNMSTPPQPNSPVFHDMV
GVVGLWRRGELASYPAGRLLRPRQPGLGDLTLRVNGGRVALNLACAIPFSTRAA
QPSAPDRLTPDLGAKLPLGDLLLRDEDGALLARVPQALYQDYWTNHGIVDLPLL
REPRGSLTLSSELAEWREQDWVTQSDASNLYLEAPDRRHGRFFPESIALRSYFRG
EARARPDIPHRIEGMGLVGVESRQDGDAAEWRLTGLRPGPARIVLDDGAEAIPLR
VLPDDWALDDATVEEVDYAFLYRHVMAYYELVYPFMSDKVFSLADRCKCETY
ARLMWQMCDPQNRNKSYYMPSTRELSAPKARLFLKYLAHVEGQARLQAPPPA
GPARIESKAQLAAELRKAVDLELSVMLQYLYAAYSIPNYAQGQQRVRDGAWTA
EQLQLACGSGDRRRDGGIRAALLEIAHEEMIHYLVVNNLLMALGEPFYAGVPLM
GEAARQAFGLDTEFALEPFSESTLARFVRLEWPHFIPAPGKSIADCYAAIRQAFLD
LPDLFGGEAAGKRGGEHHLFLNELTNRAHPGYQLEVFDRDSALFGIAFVTDQGE
GGALDSPHYEHSHFQRLREMSARIMAQSAPFEPALPALRNPVLDESPGCQRVAD
GRARALMALYQGVYELMFAMMAQHFAVKPLGSLRRSRLMNAAIDLMTGLLRP
LSCALMNLPSGIAGRTAGPPLPGPVDTRSYDDYALGCRMLARRCERLLEQASML
EPGWLPDAQMELLDFYRRQMLDLACGKLSREA (SEQ ID NO:17)

The amino acid sequence corresponding to VioE is shown in SEQ ID NO:18 below:

MENREPPLLPARWSSAYVSYWSPMLPDDQLTSGYCWFDYERDICRIDGLFNPWS
ERDTGYRLWMSEVGNAASGRTWKQKVAYGRERTALGEQLCERPLDDETGPFAE
LFLPRDVLRRLGARHIGRRVVLGREADGWRYQRPGKGPSTLYLDAASGTPLRM
VTGDEASRASLRDFPNVSEAEIPDAVFAAKR (SEQ ID NO:18)

The amino acid sequence corresponding to VioC is shown in SEQ ID NO:19 below:

KRAIIVGGGLAGGLTAIYLAKRGYEVHVVEKRGDPLRDLSSYVDVVSSRAIGVS
MTVRGIKSVLAAGIPRAELDACGEPIVAMAFSVGGQYRMRELKPLEDFRPLSLNR
AAFQKLLNKYANLAGVRYYFEHKCLDVDLDGKSVLIQGKDGQPQRLQGDMIIG
ADGAHSAVRQAMQSGLRRFEFQQTFFRHGYKTLVLPDAQALGYRKDTLYFFGM
DSGGLFAGRAATIPDGSVSIAVCLPYSGSPSLTTTDEPTMRAFFDRYFGGLPRDAR
DEMLRQFLAKPSNDLINVRSSTFHYKGNVLLLGDAAHATAPFLGQGMNMALED
ARTFVELLDRHQGDQDKAFPEFTELRKVQADAMQDMARANYDVLSCSNPIFFM
RARYTRYMHSKFPGLYPPDMAEKLYFTSEPYDRLQQIQRKQNVWYKIGRVN
(SEQ ID NO:19)

## Claims

1. A method for recovering a target product based on a synthetic organelle, comprising steps of:
a. constructing a synthetic organelle in a host;
b. using the synthetic organelle to collect a target product;
c. recovering the synthetic organelle containing the target product;
d. releasing the target product from the synthetic organelle containing the target product, thereby obtaining the target product.

2. The method according to claim 1, wherein the host includes a cell-free expression system and a cell host, preferably a prokaryotic cell host, more preferably *Escherichia coli*.

3. The method according to claim 1, wherein the synthetic organelle is an artificially designed compartmentalized structure,
preferably, wherein the synthetic organelle is a membraneless organelle, which is a biomolecular condensate formed in the host by polypeptides and/or proteins and/or DNAs and/or RNAs and/or nucleic acid-protein complexes,
more preferably, wherein the synthetic organelle is constructed from biomacromolecular components selected from the group consisting of: nucleotide sequences with 10-1,000 consecutive repeats of repeat units, such as CAG triplets, CCUG, GGGAA, and GGGGCC; amino acid sequences with 3-500 consecutive repeats, such as repeats of resilin-like peptides (RLP); intrinsically disordered regions, such as FUS protein, and ParB protein; polymerization of multivalent biomacromolecules, such as PTB protein with UCUCU sequence repeats; artificially designed binary protein two-dimensional structures; and artificially designed binary RNA two-dimensional structures.

4. The method according to claim 1, wherein the target product is one or more substances having a diameter in the range of 0.1-500 nanometers,
preferably, wherein the target product is biologically synthesized, and more preferably, the biological synthesis route of at least one component of the target product is encoded by a heterologous DNA, including: heterologously biosynthesized small molecules, such as deoxyviolacein; recombinantly expressed polypeptides, proteins, RNA, and DNA, such as green fluorescent protein, red fluorescent protein, T4 DNA ligase, and 16S rRNA; and multi-component complexes, such as 30S ribosomal subunits, and CRISPR/Cas-RNA complexes.

5. The method according to any one of claims 1 to 4, wherein the collection method in step b is non-specific and/or specific binding, and the target product originates from outside the synthetic organelle and/or is generated within the synthetic organelle,
preferably, wherein the synthetic organelle has a receptor, and the target product has an attached ligand, such that the synthetic organelle can specifically bind the target product,
preferably, wherein the receptor/ligand pair includes: complementary nucleic acid molecule pairs; capsid protein of RNA phage such as MS2, PP7, or Qbeta and translation operon RNA hairpin; protein N and boxB RNA hairpin of DNA phage such as λ, 21, or P22; CRISPR RNA hairpin and Cas6 protein of bacteria such as *Pseudomonas aeruginosa*; two segments of split protein, such as split green fluorescent protein, alpha and omega fragments of beta-galactosidase, and split T7 RNA polymerase; and inducible dimerized or polymerized monomers, such as light-controlled polymerized green fluorescent protein Dronpa.

6. The method according to any one of claims 1 to 5, wherein step c comprises separation, recovery, and purification of the synthetic organelle, preferably comprising steps of:
c1. collecting and lysing the host;
c2. separating the pellet containing the synthetic organelle, preferably, wherein the separation method is centrifugation and/or filtration;
c3. optionally, washing the pellet to remove impurities, thereby obtaining a purer synthetic organelle.

7. The method according to any one of claims 1 to 5, wherein step d comprises separation, recovery, and purification of the target product, preferably comprising steps of:
d1. placing the synthetic organelle in an eluent and inducing the synthetic organelle to release the target product, preferably, wherein the induction method is a cleavage reaction, by using a catalyst to cleave the connection site between the ligand and the target substance, said catalyst being for example nickel ions, imidazole-inducible dCsy4, factor Xa, enterokinase, dithiothreitol (DTT), thrombin, or TEV protease;
d2. separating the pellet containing the synthetic organelle to obtain the eluent containing the target product, preferably, wherein the separation method is centrifugation and/or filtration;
d3. optionally, removing impurities from the eluent to obtain the target substance with higher purity.

8. A DNA construct comprising nucleotide sequence 1 that guides the host to construct the synthetic organelle defined in the method according to any one of claims 1 to 7, and/or nucleotide sequence 2 that guides and regulates the generation of the target product defined in the method according to any one of claims 1 to 7,
preferably, wherein the DNA construct is composed of nucleotide sequences 1 and 2 and a plasmid vector, said plasmid vector being for example pACYC184, pACYC177, pET28a(+), pET28b(+), pET-5a(+), pET43.1a, pET-37b(+), pCDFDuet-1, pCOLADuet-1, pRSFDuet-1, pETDuet-1, pUC57, pUC19, pBAD, pBluescript II SK(+), pTrcHis C, pTrcHis A, pTrcHis2C, pBV221, pQE-70, pCold III, pRSET-CFP, pRSET-BFP, pGFPuv, pKD46, pKD4, pTYB1, PinPoint Xa-2, pTWIN1, pRSET C, pSB1C3, pSB3C5, pSB4C5, or pSB4K5.

9. An expression system comprising the DNA construct according to claim 8,
preferably, wherein the expression system is a system that simultaneously constructs the synthetic organelle and generates the target product in a prokaryotic cell,
more preferably, wherein the expression system is an *E. coli* expression system.

## Amended claims

### Amended claims under Art. 19.1 PCT

1. A method for recovering a target product based on a synthetic organelle, wherein the synthetic organelle is capable of collecting and releasing the target product and is isolatable and recoverable; the method comprising steps of:
a. using the synthetic organelle to collect the target product;
b. recovering the synthetic organelle containing the target product;
c. releasing the target product from the synthetic organelle containing the target product;
d. separating the synthetic organelle and obtaining the target product.

2. The method according to claim 1, wherein the synthetic organelle is an artificially designed compartmentalized structure having the property of spatial independence and being isolatable and recoverable,
preferably, wherein the synthetic organelle is a membraneless organelle, which is a biomolecular condensate formed in the host by polypeptides and/or proteins and/or DNAs and/or RNAs and/or nucleic acid-protein complexes.

3. The method according to any one of claims 1 to 2, wherein the synthetic organelle is constructed in a host, and the host includes a cell-free expression systems and a cell host, preferably a prokaryotic cell host, more preferably *E. coli*.

4. The method according to any one of claims 1 to 3, wherein the method has universality, the synthetic organelle can be constructed in diverse ways and/or from diverse components, including one or more of the following ways: formed by exogenous biomolecules produced by the host; formed by exogenous molecules combined with endogenous molecules of the host; or obtained by modification of original endogenous organelles or cytoskeleton structures of the host;
preferably, wherein the synthetic organelle is constructed from biomacromolecular components selected from the group consisting of: nucleotide sequences with 10-1,000 consecutive repeats of repeat units, such as CAG triplets, CCUG, GGGAA, and GGGGCC; amino acid sequences with 3-500 consecutive repeats, such as repeats of resilin-like peptides (RLP); intrinsically disordered regions, such as FUS protein, and ParB protein; polymerization of multivalent biomacromolecules, such as PTB protein with UCUCU sequence repeats; artificially designed binary protein two-dimensional structures; and artificially designed binary RNA two-dimensional structures.

5. The method according to claim 1, wherein the target product is one or more substances having a diameter in the range of 0.1-500 nanometers,
preferably, wherein the target product is biologically synthesized, and more preferably, the biological synthesis route of at least one component of the target product is encoded by a heterologous DNA, including: heterologously biosynthesized small molecules, such as deoxyviolacein; recombinantly expressed polypeptides, proteins, RNA, and DNA, such as green fluorescent protein, red fluorescent protein, T4 DNA ligase, and 16S rRNA; and multi-component complexes, such as 30S ribosomal subunits, and CRISPR/Cas-RNA complexes.

6. The method according to any one of claims 1 to 5, wherein in step a, the target product originates from external environment of the synthetic organelle and/or is generated within the synthetic organelle.

7. The method according to any one of claims 1 to 6, wherein the collection method in step a is non-specific and/or specific binding, achieved by one or more of the following:
1) utilizing physicochemical properties, preferably non-specific diffusion and adsorption; or
2) utilizing biomacromolecular interactions, preferably through high-affinity ligand and/or receptor pairs, more preferably, wherein the synthetic organelle has a receptor, the target product has an attached ligand, such that the synthetic organelle can specifically bind the target product.

8. The method according to any one of claims 1 to 7, wherein the method has universality, and selects different receptor/ligand pairs as needed,
preferably, wherein the receptor/ligand pairs include: complementary nucleic acid molecule pairs; capsid protein of RNA phage such as MS2, PP7, or Qbeta and translation operon RNA hairpin; protein N and boxB RNA hairpin of DNA phage such as λ, 21, or P22; CRISPR RNA hairpin and Cas6 protein of bacteria such as *Pseudomonas aeruginosa*; two segments of split protein, such as split green fluorescent protein, alpha and omega fragments of beta-galactosidase, and split T7 RNA polymerase; and inducible dimerized or polymerized monomers, such as light-controlled polymerized green fluorescent protein Dronpa.

9. The method according to any one of claims 1 to 8, wherein step b comprises separation, recovery, and purification of the synthetic organelle, preferably comprising steps of:
b1. collecting and lysing the host;
b2. separating the pellet containing the synthetic organelle, preferably by centrifugation and/or filtration;
b3. optionally, washing the pellet to remove impurities, thereby obtaining a purer synthetic organelle.

10. The method according to any one of claims 1 to 9, wherein step c comprises placing the synthetic organelle in an eluent and allowing the synthetic organelle to release the target product, by one or more of the following:
1) using an eluent with high affinity, for example, using methanol for deoxyviolacein;
2) changing the affinity of the synthetic organelle, for example using a receptor and ligand pair sensitive to temperature, light, or pH;
3) disrupting the connection between the target product and a ligand so that the synthetic organelle loses affinity for the target product, thereby inducing the synthetic organelle to efficiently release the target product without a ligand tag, preferably wherein the induction method is a cleavage reaction by using a catalyst to cleave the connection site between the ligand and the target product, the catalyst being for example nickel ions, imidazole-inducible dCsy4, factor Xa, enterokinase, dithiothreitol (DTT), thrombin, or TEV protease; or
4) allowing the target product to spontaneously diffuse into the eluent until equilibrium.

11. The method according to any one of claims 1 to 10, wherein step d comprises separation, recovery and purification of the target product, preferably comprising steps of:
d1. separating the pellet containing the synthetic organelle to obtain the eluent containing the target product, preferably by centrifugation and/or filtration;
d2. optionally, removing impurities from the eluent to obtain the target substance with higher purity.

12. A DNA construct comprising nucleotide sequence 1 that guides the host to construct the synthetic organelle defined in the method according to any one of claims 1 to 11, and/or nucleotide sequence 2 that guides and regulates the generation of the target product defined in the method according to any one of claims 1 to 11,
preferably, wherein the DNA construct is composed of nucleotide sequences 1 and 2 and a plasmid vector, said plasmid vector being for example pACYC184, pACYC177, pET28a(+), pET28b(+), pET-5a(+), pET43.1a, pET-37b(+), pCDFDuet-1, pCOLADuet-1, pRSFDuet-1, pETDuet-1, pUC57, pUC19, pBAD, pBluescript II SK(+), pTrcHis C, pTrcHis A, pTrcHis2C, pBV221, pQE-70, pCold III, pRSET-CFP, pRSET-BFP, pGFPuv, pKD46, pKD4, pTYB1, PinPoint Xa-2, pTWIN1, pRSET C, pSB1C3, pSB3C5, pSB4C5, or pSB4K5.

13. An expression system comprising the DNA construct according to claim 12, including one or more of the following:
1) a system that simultaneously constructs the synthetic organelle and generates the target product in a prokaryotic cell, preferably an *E. coli* expression system;
2) a combination of multiple systems that respectively construct the synthetic organelle and generate the target product, preferably, wherein the synthetic organelle is constructed in *E. coli* and the target product is synthesized in a cell-free system.

14. The method according to any one of claims 1 to 11, wherein the method has universality; and adopts substantially the same processes for different synthetic organelles and/or target products and/or eluent properties, preferably enabling high-throughput parallel processing.
